(19) Europäisches Patentamt
European Patent Office
Office européen des brevets

(11) **EP 3 031 472 A1**

(12) **EUROPEAN PATENT APPLICATION**

(43) Date of publication:
15.06.2016 Bulletin 2016/24

(51) Int Cl.:
*A61K 51/06* (2006.01)   *A61P 35/00* (2006.01)
*A61K 51/12* (2006.01)

(21) Application number: 15194450.1

(22) Date of filing: 25.10.2001

(84) Designated Contracting States:
**AT BE CH CY DE DK ES FI FR GB GR IE IT LI LU MC NL PT SE TR**

(30) Priority: 25.10.2000 AU PP098300

(62) Document number(s) of the earlier application(s) in accordance with Art. 76 EPC:
**01978015.4 / 1 333 866**

(71) Applicant: **SIRTEX MEDICAL LIMITED North Ryde New South Wales 2113 (AU)**

(72) Inventor: **GRAY, Bruce, Nathaniel Claremont, Western Australia 6010 (AU)**

(74) Representative: **Lee, Nicholas John et al Kilburn & Strode LLP 20 Red Lion Street London WC1R 4PJ (GB)**

Remarks:
•This application was filed on 13-11-2015 as a divisional application to the application mentioned under INID code 62.
•Claims filed after the date of filing of the application / after the date of receipt of the divisional application (Rule 68(4) EPC)

(54) **POLYMER BASED RADIONUCLIDE CONTAINING PARTICULATE MATERIAL**

(57) The invention relates to a particulate material having a diameter in the range of from 5 to 200 microns comprising a polymeric matrix and stably incorporated radionuclide, processes for its production and a method of radiation therapy utilising the particulate material.

Figure 1

**Description**

**FIELD OF THE INVENTION**

This invention relates to a particulate material that comprises a polymer, particularly a polymer and a radionuclide, to a method for the production thereof, and to methods for the use of this particulate material.

In one particular aspect, this invention relates to microspheres which comprise a polymer and a radionuclide such as radioactive yttrium, and to the use of these microspheres in the treatment of cancer in humans and other mammals.

The particulate material of this invention is designed to be administered into the arterial blood supply of an organ to be treated, whereby it becomes entrapped in the small blood vessels of the target organ and irradiates it. An alternate form of administration is to inject the polymer based particulate material directly into the target organ or a solid tumour to be treated.

The particulate material of the present invention therefore has utility in the treatment of various forms of cancer and tumours, but particularly in the treatment of primary and secondary cancer of the liver and the brain. It is to be understood that the particulate material of the invention is not limited to radioactive microspheres, but may be extended to other radioactive polymeric particles which are suitable for use in the treatment methods described herein.

**BACKGROUND OF THE INVENTION**

Many previous attempts have been made to locally administer radioactive materials to patients with cancer as a form of therapy. In some of these, the radioactive materials have been incorporated into small particles, seeds, wires and similar related configurations that can be directly implanted into the cancer. When radioactive particles are administered into the blood supply of the target organ, the technique has become known as Selective Internal Radiation Therapy (SIRT). Generally, the main form of application of SIRT has been its use to treat cancers in the liver.

There are many potential advantages of SIRT over conventional, external beam radiotherapy. Firstly, the radiation is delivered preferentially to the cancer within the target organ. Secondly, the radiation is slowly and continually delivered as the radionuclide decays. Thirdly, by manipulating the arterial blood supply with vasoactive substances (such as Angiotensin-2), it is possible to enhance the percentage of radioactive particles that go to the cancerous part of the organ, as opposed to the healthy normal tissues. This has the effect of preferentially increasing the radiation dose to the cancer while maintaining the radiation dose to the normal tissues at a lower level (Burton, M.A. et al.; Effect of Angiotensin-2 on blood flow in the transplanted sheep squamous cell carcinoma. Europ. J. Cancer Clin. Oncol. 1988, 24(8):1373-1376).

When microspheres or other small particles are administered into the arterial blood supply of a target organ, it is desirable to have them of a size, shape and density that results in the optimal homogeneous distribution within the target organ. If the microspheres or small particles do not distribute evenly, and as a function of the absolute arterial blood flow, then they may accumulate in excessive numbers in some areas and cause focal areas of excessive radiation. It has been shown that microspheres of approximately 25-50 micron in diameter have the best distribution characteristics when administered into the arterial circulation of the liver (Meade, V. et al.; Distribution of different sized microspheres in experimental hepatic tumours. Europ. J. Cancer & Clin. Oncol. 1987, 23:23-41).

If the particles are too dense or heavy, then they will not distribute evenly in the target organ and will accumulate in excessive concentrations in areas that do not contain the cancer. It has been shown that solid, heavy microspheres distribute poorly within the parenchyma of the liver when injected into the arterial supply of the liver. This, in turn, decreases the effective radiation reaching the cancer in the target organ, which decreases the ability of the radioactive microspheres to kill the tumour cells. In contrast, lighter microspheres with a specific gravity of the order of 2.0 distribute well within the liver (Burton, M.A. et al.; Selective International Radiation Therapy; Distribution of radiation in the liver. Europ. J. Cancer Clin. Oncol. 1989, 25:1487-1491).

For radioactive particulate material to be used successfully for the treatment of cancer, the radiation emitted should be of high energy and short range. This ensures that the energy emitted will be deposited into the tissues immediately around the particulate material and not into tissues which are not the target of the radiation treatment. In this treatment mode, it is desirable to have high energy but short penetration beta-radiation which will confine the radiation effects to the immediate vicinity of the particulate material. There are many radionuclides that can be incorporated into microspheres that can be used for SIRT. Of particular suitability for use in this form of treatment is the unstable isotope of yttrium (Y-90).

Yttrium-90 decays with a half life of 64 hours, while emitting a high energy pure beta radiation. However, other radionuclides may also be used in place of yttrium-90 of which the isotopes of holmium, samarium, iodine, iridium, phosphorus, rhenium are some examples.

Ceramic particles have been produced that are either coated with or contain radionuclides. However, the presence of other radioactive substances that are not required for the radiation treatment of the target tissue, has then unwanted and deleterious radiation effects may occur. It is therefore desirable to have particulate material of such a composition that it only contains the single desired radionuclide.

In the earliest clinical use of yttrium-90 containing microspheres, the yttrium was incorporated into a polymeric matrix that was formulated into microspheres. While these microspheres were of an appropriate density to ensure good distribution characteristics in the liver, there were several instances in which the yttrium-90 leached from the microspheres and caused inappropriate radiation of other tissues. Attempts to incorporate other radionuclides such as holmium into resin or polymer based materials have resulted in leaching of the radionuclide and this has resulted in severe consequences for the patients that have been treated with the product.

In one attempt to overcome the problem of leaching, a radioactive microsphere comprising a biologically compatible glass material containing a beta- or gamma-radiation emitting radioisotope such as yttrium-90 distributed throughout the glass, has been developed (International Patent Publication No. WO 86/03124). These microspheres are solid glass and contain the element yttrium-89 that can be activated to the radionuclide yttrium-90 by placing the microspheres in a neutron beam. These glass microspheres have several disadvantages including being of a higher specific gravity than is desirable and containing other elements such as alumina and silica which are activated to undesirable radionuclides when placed in a neutron beam.

Another approach has been focussed on the use of small hollow or cup-shaped ceramic particles or microspheres, wherein the ceramic base material consists or comprises yttria or the like (International Patent Publication No. WO 95/19841). These microspheres were developed to overcome the problem of high density associated with the solid glass microspheres described in International Patent Publication No. WO86/03124.

## SUMMARY OF THE INVENTION

In one aspect the present invention provides a particulate material having a diameter in the range of from 5 to 200 microns comprising a polymeric matrix and a stably incorporated radionuclide.

In another aspect, the invention provides a process for the production of a particulate material having a diameter in the range of from 5 to 200 microns comprising the step of combining a polymeric matrix and a radionuclide for a time and under conditions sufficient to stably incorporate the radionuclide in the matrix to produce a particulate material having a diameter in the range of from 5 to 200 microns.

In another aspect, the present invention provides a method of radiation therapy of a patient, which comprises administration to the patient of a particulate material having a diameter in the range of from 5 to 200 microns comprising a polymeric matrix and a stably incorporated radionuclide.

The present invention also provides for the use of particulate material having a diameter in the range of from 5 to 200 microns comprising a polymeric matrix and a stably incorporated radionuclide in the radiation therapy of a patient.

## DETAILED DESCRIPTION OF THE INVENTION

As used herein, references to the radionuclide being stably incorporated into particulate material or polymeric matrix are to be understood as referring to incorporation of the radionuclide so that it does not substantially leach out of the particulate material under physiological conditions such as in the patient or in storage. In a preferred embodiment the radionuclide is incorporated by precipitation into a polymeric matrix.

The leaching of radionuclides from the polymeric matrix can cause non-specific radiation of the patient and damage surrounding tissue. Preferably the amount of leaching is less than 5%, more preferably less than 4%, 3%, 2%, 1% or 0.4%. One method of assessing leaching is by adjusting a sample to pH 7.0 and agitating in a water bath at 37°C for 20 minutes. A 100 $\mu$L sample is counted for beta emission in a Geiger-Müller counter. Another representative 100 $\mu$L sample is filtered through a 0.22 $\mu$m filter and the filtrate counted for beta emission in the Geiger-Müller counter. The per cent unbound radionuclide is calculated by:

$$\frac{FiltrateCount}{SampleCount} \times 100 = \% \, UnboundRadionuclide$$

The radionuclide can be stably incorporated into the polymeric matrix by precipitating it as an insoluble salt. Where the radionuclide used is yttrium-90 the yttrium is preferably precipitated as a phosphate salt. However the present invention also extends to precipitation of the radionuclide as other insoluble salts including, for example, carbonate and bicarbonate salts. The radionuclide which is incorporated into the polymeric matrix in accordance with the present invention is preferably yttrium-90, but may also be any other suitable radionuclide which can be precipitated in solution, of which the isotopes of holmium, samarium, iodine, phosphorous, iridium and rhenium are some examples.

In a preferred embodiment the particulate material is a microsphere. The term microsphere is used in this specification as an example of a particulate material, it is not intended to limit the invention to microspheres, as the person skilled in the art will appreciate that the shape of the particulate material while preferably without sharp edges or points that could damage the patients arteries or catch in unintended locations, is not limited to spheres. Nor should the term microsphere be limited to spheres. Preferably the particulate material is substantially spherical, but need not be regular or symmetrical in shape.

In a preferred embodiment the polymeric matrix is partially cross linked. Preferably there is about 1% to about 20% cross linking, preferably about 2% to 10% cross linking and more preferably about 4% cross linking.

In particular, the present invention provides a particulate material as described above in which the polymeric matrix is an ion exchange resin, particularly a cation exchange resin. Preferably the ion exchange resin comprises a partially cross linked aliphatic polymer, including polystyrene. One particularly preferred cation exchange resin is the styrene/divinylbenzene copolymer resin commercially available under the trade name Aminex 50W-X4 (Biorad, Hercules, CA). However, there are many other commercially available cation exchange resins which are suitable.

When small particles are administered into the arterial blood supply of a target organ, it is desirable to have them of a size, shape and density that results in the optimal homogeneous distribution within the target organ. If the small particles do not distribute evenly then they may accumulate in excessive numbers in some areas and cause focal areas of excessive radiation. The particulate material is preferably low density, more particularly a density below 3.0 g/cc, even more preferably below 2.8g/cc, 2.5g/cc, 2.3g/cc, 2.2g/cc or 2.0 g/cc. The ideal particle for injection into the blood stream would have a very narrow size range with a SD of less than 5%, so as to assist in even distribution of the microspheres within the target organ, particularly within the liver and would be sized in the range 5-200 micron preferably 15-100 micron and preferably 20-50 micron, and most preferably 30-35 micron.

It is also desirable to have the particulate material manufactured so that the suspending solution has a pH less than 9. If the pH is greater than 9 then this may result in irritation of the blood vessels when the suspension is injected into the artery or target organ. Preferably the pH is less than 8.5 or 8.0 and more preferably less than 7.5.

The present invention particularly provides a method for the production of a radioactive particulate material comprising a polymeric matrix as described above, characterised by the steps of:

(i) absorbing a radionuclide onto an ion-exchange resin particulate material having a diameter in the range of 20 to 50 microns and a specific gravity of less than 2.5; and

(ii) precipitating the radionuclide as an insoluble salt to stably incorporate the radionuclide into the particulate material.

In a preferred embodiment, the method of the present invention is carried out by firstly irradiating yttria (yttrium oxide) in a neutron beam to activate yttria to the isotope yttrium-90. The yttrium-90 oxide is then solubilised, for example as yttrium-90 sulphate solution. The ion exchange resin is preferably provided in the form of an aqueous slurry of microspheres of ion exchange resin having a particle size 30 to 35 microns, and the yttrium-90 sulphate solution is added to the slurry to absorb the yttrium-90 into the ion exchange resin microspheres. Subsequently, the yttrium-90 is precipitated as a phosphate salt, for example by addition of tri-sodium phosphate solution, to stably incorporate the yttrium-90 into the microspheres. The particulate material may be combined with a solution of the radionuclide or the salt of the radionuclide may be combined with the particulate matter, in a solution suitable for solubilising the radionuclide.

Alternate sources of yttrium-90 may be used in the production of these microspheres. For example, a highly pure source of yttrium-90 may be obtained by extracting yttrium-90 from a parent nuclide and using this extracted yttrium-90 as the source of the soluble yttrium salt that is then incorporated into the polymeric matrix of the microspheres.

In order to decrease the pH of the suspension containing the microspheres for injection into patients the microspheres may be washed to remove any un-precipitated or loosely adherent radionuclide. The present invention provides a suspension of the required pH by precipitating the yttrium with a tri-sodium phosphate solution at a concentration containing at least a threefold excess of phosphate ion, but not exceeding a 30-fold excess of phosphate ion, and then washing the microspheres with de-ionised water. Another approach which ensures that the pH of the microsphere suspension is in the desired range is to wash the resin with a phosphate buffer solution of the desired pH.

The present invention also provides a method of radiation therapy of a human or other mammalian patient that comprises administration to the patient of particulate material as described above. The person skilled in the art will appreciate the administration may be by any suitable means and preferably by delivery to the relevant artery. For example in treating liver cancer, administration is preferably by laparotomy to expose the hepatic artery or by insertion of a catheter into the hepatic artery via the femoral, or brachial artery. Pre or co-administration of another agent may prepare the tumour for receipt of the particulate material, for example a vasioactive substance, such as angiotension-2 to redirect arterial blood flow into the tumour. Delivery of the particulate matter may be by single or multiple doses, until the desired level of radiation is reached.

Throughout this specification, unless the context requires otherwise, the word "comprise", and or variations such as "comprises" or "comprising", will be understood to imply the inclusion of a stated integer or step or group of integers or steps but not the exclusion of any other integer or step or group of integers or steps.

Further features of the present invention are more fully described in the following Examples. It is to be understood, however, that this detailed description is included solely for the purposes of exemplifying the present invention, and should not be understood in any way as a restriction on the broad description of the invention as set out above.

EXAMPLE 1

Yttrium (90Y) labelled microspheres are made in the form of a sterile, pyrogen free suspension of resin beads labelled with yttrium (90Y) phosphate. The resin beads consist of sulphuric acid groups attached to a styrene divinylbenzene copolymer lattice.

Yttrium oxide is irradiated to produce yttrium-90 from the nuclear reaction Y-89 (n, $\gamma$) Y-90. Yttrium-90 has a half life of 64 hours. The yttrium (90Y) oxide is then dissolved in 0.1M sulphuric acid with gentle heating and stirring to form a clear, colourless solution of yttrium (90Y) sulphate.

Symmetrical microspheres of ion exchange resin (Aminex 50W-X4 cation exchange resin; supplied by 'Bio-Rad Cat # 1474313') with a diameter of approximately 30 to 35 microns are added to water (Water for Injections BP) to form a slurry that is then transferred into a reaction vessel. Yttrium (90Y) sulphate solution is added to the reaction vessel and the mixture stirred at a speed sufficient to ensure homogeneity to absorb the yttrium (90Y) solution into the resin-based microspheres. Tri-sodium phosphate solution (1.25% w/v) is then added to the reaction vessel with further stirring to precipitate the radionuclide as yttrium (90Y) phosphate.

The microspheres are then washed with a phosphate buffer solution until the pH of the wash solution is less than 9 and preferable less than 8.5. Following washing of the microspheres with water (Water for Injection BP), the microspheres are resuspended and diluted (if necessary) with water (Water for Injections BP) to give a light brown suspension having an activity of 3000 MBq □10%.

The resin-based yttrium microspheres produced by the above method have 0.01-0.4% unbound or unprecipitated 90Y when tested in the following leaching test:

A 5 $\mu$L sample is diluted with water to 5 mL, adjusted to pH 7.0 and agitated in a water bath at 37°C for 20 minutes. A 100 $\mu$L sample is counted for beta emission in a Geiger-Müller counter. Another representative 100 $\mu$L sample is filtered through a 0.22 $\mu$m filter and the filtrate counted for beta emission in the Geiger-Müller counter. The per cent unbound 90Y is calculated by:

$$\frac{FiltrateCount}{SampleCount}\ X\ 100 = \%\ Unbound\ ^{90}Y$$

EXAMPLE 2

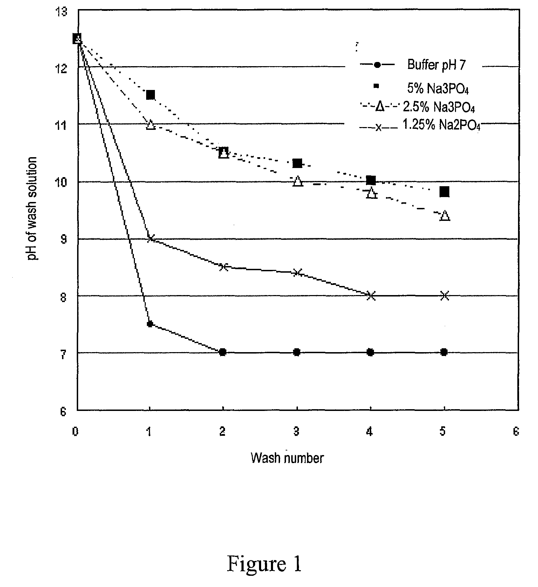

The effect of phosphate concentration in the precipitation solution, and the effects of washing with phosphate buffer on the pH of a microsphere suspension are shown in the attached Figure 1 which sets out the results of a number of experiments.

EXAMPLE 3

The technique of Selective Internal Radiation Therapy (SIRT) has been described above. It involves either a laparotomy to expose the hepatic arterial circulation or the insertion of a catheter into the hepatic artery via the femoral, brachial or other suitable artery. This may be followed by the infusion of Angiotensin-2 into the hepatic artery to redirect arterial blood to flow into the metastatic tumour component of the liver and away from the normal parenchyma. This is followed by embolisation of resin based yttrium-90 containing microspheres (produced in accordance with Example 1) into the arterial circulation so that they become lodged in the microcirculation of the tumour. Repeated injections of microspheres are made until the desired radiation level in the normal liver parenchyma is reached. By way of example, an amount of yttrium-90 activity that will result in an inferred radiation dose to the normal liver of approximately 80 Gy may be delivered. Because the radiation from SIRT is delivered as a series of discrete point sources, the dose of 80 Gy is an average dose with many normal liver parenchymal cells receiving much less than that dose.

The measurement of tumour response by objective parameters including reduction in tumour volume and serial estimations of serum carcino-embryonic antigen (CEA) levels, is an acceptable index of the ability of the treatment to alter the biological behaviour of the tumour.

Certain aspects of the invention are set out in the following numbered paragraphs.

1. In one aspect, the invention provides a particulate material having a diameter in the range of from 5 to 200 microns comprising a polymeric matrix and stably incorporated radionuclide.

2. In the particulate material of paragraph 1, the radionuclide may be incorporated by precipitation.

3. In the particulate material of paragraph 1 the polymeric matrix may be partially cross linked.

4. In the particulate material of paragraph 3 the polymeric matrix may comprise from about 1% to about 20% cross linking.

5. In the particulate material of paragraph 4 the polymeric matrix may comprise about 4% cross linking.

6. In the particulate material of paragraph 1 the polymeric matrix may be an ion exchange resin.

7. In the particulate material of paragraph 6 the polymeric matrix may be a cation exchange resin.

8. In the particulate material of paragraph 6 the ion exchange resin may comprise a partially cross linked aliphatic polymer.

9. In the particulate material of paragraph 6 the ion exchange resin may comprise a partially cross linked polystyrene.

10. In the particulate material of paragraph 9 the ion exchange resin may comprise polystyrene partially cross linked with divinyl benzene.

11. In the particulate material of paragraph 1, the radionuclide may be an isotope of yttrium, holmium, samarium, iodine, phosphorus, iridium or rhenium.

12. In the particulate material of paragraph 1, the radionuclide may be yttrium-90.

13. In the particulate material of paragraph 1 may be a microsphere.

14. In another aspect, the invention provides a particulate material having a diameter in the range of from 30 to 35 microns comprising a copolymer comprised of styrene and divinyl benzene and precipitated yttrium-90.

15. In another aspect, the invention provides a process for the production of a particulate material of paragraph 1 comprising the step of combining a polymeric matrix and a radionuclide in solution for a time and under conditions sufficient to stably incorporate the radionuclide in the matrix to produce a particulate material having a diameter in the range of from 5 to 200 microns.

16. In the process of paragraph 15 the radionuclide may be stably incorporated by precipitation into the polymeric matrix.

17. In the process according to claim 15 wherein the radionuclide may be yttrium-90.

18. In another aspect, the invention provides a method of radiation therapy of a patient, which comprises administration to the patient of a particulate material having a diameter in the range of from 5 to 200 microns comprising a polymeric matrix and a stably incorporated radionuclide.

19. In the method of paragraph 18 the radionuclide may be yttrium-90.

20. In the method of paragraph 18 the radiation therapy may comprise treatment of a primary or secondary liver cancer.

21. In another aspect, the invention provides use of particulate material having a diameter in the range of from 5 to 200 microns comprising a polymeric matrix and a stably incorporated radionuclide in radiation therapy of a patient.

22. In the use of paragraph 21 the radionuclide may be yttrium-90.

23. In the use of paragraph 21 the radiation therapy may comprise treatment of a primary or secondary liver cancer.

**Claims**

1. A particulate material having a diameter in the range of from 5 to 200 microns comprising a polymeric matrix and stably incorporated radionuclide.

2. The particulate material according to claim 1 wherein the radionuclide is incorporated by precipitation.

3. The particulate material according to claim 1 wherein the polymeric matrix is partially cross linked, optionally wherein the polymeric matrix comprises from about 1% to about 20% or about 4% cross linking.

4. The particulate material according to claim 1 wherein the polymeric matrix is an ion exchange resin, which optionally comprises a partially cross linked aliphatic polymer or a partially cross linked polystyrene, such as polystyrene partially cross linked with divinyl benzene, or is a cation exchange resin.

5. The particulate material according to claim 1, wherein the radionuclide is an isotope of yttrium, holmium, samarium, iodine, phosphorus, iridium or rhenium.

6. The particulate material according to claim 1, wherein the radionuclide is yttrium-90.

7. The particulate material according to claim 1 being a microsphere.

8. A particulate material having a diameter in the range of from 30 to 35 microns comprising a copolymer comprised of styrene and divinyl benzene and precipitated yttrium-90.

9. A process for the production of a particulate material according to claim 1 comprising the step of combining a polymeric matrix and a radionuclide in solution for a time and under conditions sufficient to stably incorporate the radionuclide in the matrix to produce a particulate material having a diameter in the range of from 5 to 200 microns.

10. A process according to claim 9 wherein the radionuclide is stably incorporated by precipitation into the polymeric matrix.

11. A process according to claim 9 wherein the radionuclide is yttrium-90.

12. A particulate material according to claim 1 or claim 6 for use in a method of radiation therapy of a patient, which comprises administration to the patient of the particulate material.

13. A particulate material according to claim 12 wherein the radiation therapy comprises treatment of a primary or secondary liver cancer.

14. Use of particulate material according to claim 1 or claim 6 in the manufacture of a medicament for use in radiation therapy of a patient.

15. Use according to claim 14 wherein the radiation therapy comprises treatment of a primary or secondary liver cancer.

Figure 1

Europäisches
Patentamt

European
Patent Office

Office européen
des brevets

# EUROPEAN SEARCH REPORT

**Application Number**

EP 15 19 4450

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CHAMBERLAIN M N ET AL: "Hepatic metastases--a physiological approach to treatment.", THE BRITISH JOURNAL OF SURGERY OCT 1983, vol. 70, no. 10, October 1983 (1983-10), pages 596-598, XP009082540, ISSN: 0007-1323 * the whole document * | 1-15 | INV. A61K51/06 A61P35/00 A61K51/12 |
| X | WANG S-J ET AL: "Intratumoral injection of rhenium-188 microspheres into an animal model of hepatoma", JOURNAL OF NUCLEAR MEDICINE, SOCIETY OF NUCLEAR MEDICINE, RESTON, VA, US, vol. 39, no. 10, October 1998 (1998-10), pages 1752-1757, XP002337420, ISSN: 0161-5505 * the whole document * | 1-15 | |
| X | ANONYMOUS: "SIRTEX MEDICAL LIMITED PROSPECTUS", INTERNET CITATION, 1 January 2000 (2000-01-01), pages I-VIII, XP003031978, Retrieved from the Internet: URL:http://schoolofeducators.com/wp-content/uploads/2008/08/Prospectus11.pdf [retrieved on 2013-08-01] * the whole document * | 1-15 | TECHNICAL FIELDS SEARCHED (IPC)<br><br>A61K<br>A61P |

-/--

The present search report has been drawn up for all claims

| Place of search | Date of completion of the search | Examiner |
|---|---|---|
| Munich | 29 February 2016 | Estañol, Inma |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

...................................................................................................
& : member of the same patent family, corresponding
   document

EPO FORM 1503 03.82 (P04C01)

Europäisches
Patentamt

European
Patent Office

Office européen
des brevets

**EUROPEAN SEARCH REPORT**

Application Number

EP 15 19 4450

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HO S ET AL: "Clinical Evaluation of the Partition Model for Estimating Radiation Doses from Yttrium-90 Microspheres in the Treatment of Hepatic Cancer", EUROPEAN JOURNAL OF NUCLEAR MEDICINE, SPRINGER, BERLIN, HEIDELBERG, DE, vol. 24, no. 3, 1 March 1997 (1997-03-01), pages 293-298, XP002531708, ISSN: 0340-6997 * the whole document * | 1-15 | |
| X | LAU W-Y ET AL: "Treatment of inoperable hepatocellular carcinoma with intrahepatic arterial yttrium-90 microspheres: a phase I and II study", BRITISH JOURNAL OF CANCER, NATURE PUBLISHING GROUP, GB, vol. 70, 1 January 1994 (1994-01-01), pages 994-999, XP003029382, ISSN: 0007-0920 * the whole document * | 1-15 | |
| X | GRADY ET AL: "Intrahepatic aterial 90-yttrium resin spheres to treat liver cancer", INTERNATIONAL JOURNAL OF NUCLEAR MEDICINE AND BIOLOGY, PERGAMON PRESS, OXFORD, GB, vol. 5, no. 6, 1 January 1978 (1978-01-01), pages 253-254, XP026259406, ISSN: 0047-0740, DOI: 10.1016/0047-0740(78)90150-X [retrieved on 1978-01-01] * the whole document * | 1-15 | **TECHNICAL FIELDS SEARCHED (IPC)** |

-/--

The present search report has been drawn up for all claims

| Place of search | Date of completion of the search | Examiner |
|---|---|---|
| Munich | 29 February 2016 | Estañol, Inma |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 15 19 4450

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MEADE V M ET AL: "Distribution of different sized microspheres in experimental hepatic tumours", EUROPEAN JOURNAL OF CANCER AND CLINICAL ONCOLOGY, PERGAMON PRESS LTD, vol. 23, no. 1, 1 January 1987 (1987-01-01), pages 37-41, XP026204063, ISSN: 0277-5379, DOI: 10.1016/0277-5379(87)90416-0 [retrieved on 1987-01-01] * the whole document * ----- | 1-15 | |

TECHNICAL FIELDS
SEARCHED (IPC)

The present search report has been drawn up for all claims

| Place of search | Date of completion of the search | Examiner |
|---|---|---|
| Munich | 29 February 2016 | Estañol, Inma |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

........................................................................................

& : member of the same patent family, corresponding
document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 8603124 A

- WO 9519841 A

**Non-patent literature cited in the description**

- **BURTON, M.A. et al.** Effect of Angiotensin-2 on blood flow in the transplanted sheep squamous cell carcinoma. *Europ. J. Cancer Clin. Oncol.,* 1988, vol. 24 (8), 1373-1376
- **MEADE, V. et al.** Distribution of different sized microspheres in experimental hepatic tumours. *Europ. J. Cancer & Clin. Oncol.,* 1987, vol. 23, 23-41

- **BURTON, M.A. et al.** Selective International Radiation Therapy; Distribution of radiation in the liver. *Europ. J. Cancer Clin. Oncol.,* 1989, vol. 25, 1487-1491